# EUROPEAN PATENT APPLICATION

(11) **EP 4 066 733 A1**
(43) Date of publication of application: **05.10.2022**
(21) Application number: 21000093.1
(22) Date of filing: 31.03.2021
(51) Int. Cl.: A61B 5/01, A61B 5/00, A41B 13/00, A41D 11/00, A41D 13/12

(54) **BABY UNDERWEAR FOR CONTINUOUS SURFACE SKIN TEMPERATURE MEASUREMENT**

(71) Applicant: Ackermann Market Kft., 2030 Érd (HU)
(72) Inventor: The designation of the inventor has not yet been filed
(74) Representative: Giber, Janos

(57) **Abstract**

Baby underwear for measuring the continuous surface skin temperature. The baby undergarment (1) comprises an internal heat sensor (2) in at least one metal-covered snap cover (5) in direct contact with the skin above the liver area sensing the skin temperature via a directly contacting metal housing and with a related central unit (10) comprising a microprocessor (7), a power source (8) and a communication device (9). Through this central unit the internal sensing unit is connected with a data processing and communications mobile phone (6) with an alarm function wherein the internal heat sensing unit (2) has a snapping dress strap (11) in the middle on the left front side of the baby underwear (1) with a convex surface facing the baby body located in a convex snap cover (5), fastened from above to the textile strap (3) clamped between the two snap halves by the circular part (4) of the snap connector, so in that the heat sensor (2) located in the snap cover (5) is connected to a central unit (10) located in the lower part of the snapping clothing strap (11).

## Description

The subject matter of the invention is a baby underwear (baby body) for measuring the continuous surface skin temperature to recognize emergencies due to an increase or decrease of temperature of a sleeping child, composing at least one metal-clad internal heat sensor unit (2) placed inside a metal-covered snap cover (5) in a direct contact with the skin above the liver area, sensing the skin temperature via a directly contacting metal housing and furthermore comprising a related central unit (10) comprising a microprocessor (7), energy supply, a power source (8) and a communication device (9) linked to and communicating with a data processing and alerting system installed on external mobile phone (6).

Sudden Infant Death Syndrome (SIDS) is known to affect infants less than one year of age. SIDS occurs unexpectedly and quickly in seemingly healthy babies, usually stopping the baby from breathing during sleep. A major risk factor for sudden infant death is overheating or cooling of the body due to internal (low or high fever, circulatory insufficiency, etc.) or external conditions (eg excessive covering, overdressing, unreasonably high room temperature, unreasonable cap wear, low outside temperature, or insufficient one-layer dress, etc). Developing (and therefore still underdeveloped) thermoregulation of infants and faulty baby dressing and bedtime rituals have been proven to cause persistent heat stress for one in six infants, and a significant proportion of these infants are unable to maintain body temperature within optimal limits. This in itself is detrimental to health which, in the presence of additional risk factors, can lead to a life-threatening condition and, in the worst case, to cradle death. In addition, a number of acute infectious diseases are known which, with a sudden increase in nocturnal fever within a few hours, endanger the circulation and life of the baby or child.

One of the determinants of the developing and initially underdeveloped thermoregulation in infants is the immaturity of the dermal excretion of skin as a thermoregulatory system and the partial or complete absence of operating conditions. Intensive sweating is an indicator and concomitant of many life-threatening conditions (eg cradle death, acute fever attack). In early infancy, the ability or inhibition of the areas around the neck and head to evaporate is critical to the functioning of the thermoregulatory system.

Parents cover the babies with blankets and quilts to prevent them from catching a cold. During involuntary movements during the night's sleep, the neck and head, actually the babies' almost exclusive evaporating surfaces, are covered by a blanket or bedding which is both suffocating and partially or completely paralyzing the baby's heat stress protection system.

Several technologies are known for measuring a baby's temperature using heat sensors placed in the oral cavity, armpits, rectum, ear canals, forehead-skin, but typically none of them provide continuous temperature monitoring to warn during sleep or overnight of too high or too low body temperature. The object of the present invention is to provide a children's underwear (baby body) to satisfy the above need to prevent or recognize part of the occurrence of such emergencies by monitoring the temperature of the sleeping child. To solve this task, i.e. to detect emergencies related to changes in body temperature, baby underwear includes a convex metal snap cover facing the baby's body surface, which includes a built-in heat sensing unit which is also wired to a central unit built into the garment, a power source and a communication device, and is in communication with a data processing and alarm system installed on the parent's mobile phone, where both the internal heat sensor unit and the central unit are located in the strap of the baby garment.

In a preferred embodiment of the invention, the connection between the heat sensor unit (2) and the communication device (9) in the central unit (10) and the connection between the communication device (9) and the mobile telephone (6) are wireless.

As a preferred solution of the invention, wireless connection is provided by Bluetooth, preferably Bluetooth Low Energy (BLE).

As a preferred solution of the invention, rechargeable battery is used for source of energy in the internal sensor unit.

As a preferred solution of the invention, the heat sensor snap is placed on the right side of the baby's underwear above the liver area.

As a preferred solution of the invention, several heat sensors may be placed on the garment's strap containing snaps.

The solution provided by the invention shall be described in more detail by examples based on accompanying drawings.

In the drawing:

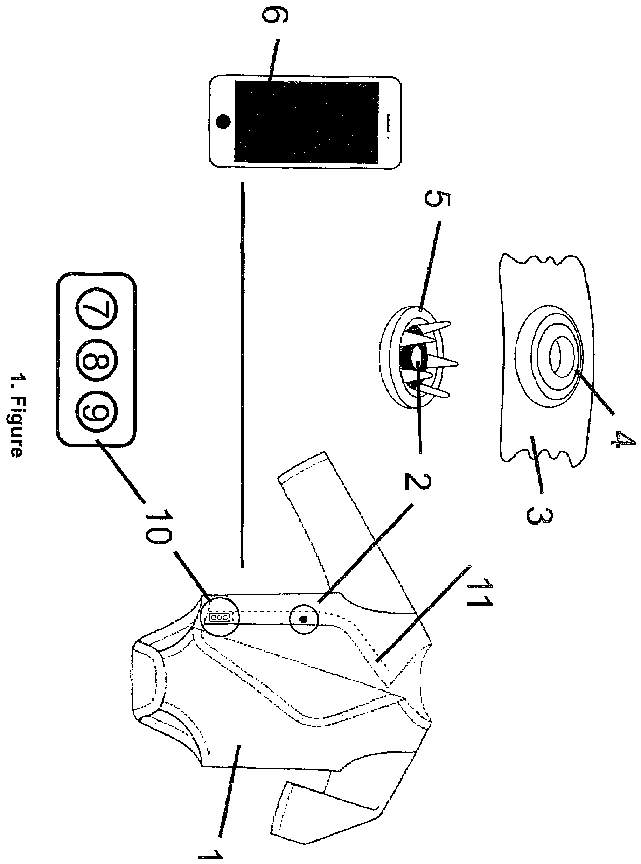

Figure 1 is the invention's schematic representation of baby underwear (baby body) and built-in heat sensor system with its central unit connected with wireless mobile telephone. Details are magnified and emergency situations due to increasing and lowering body temperatures of sleeping children shown.

Figure 1 is a schematic representation of a baby underwear (baby body) according to the present invention for recognizing emergencies related to a sleeping child, where the baby underwear (baby body) (1) is shown schematically in a plan view: baby underwear (1) in at least one snap cover (5) with a metal cover in direct contact with the skin, comprising an internal heat sensor (2) which detects the temperature of the skin through a metal cover in direct contact with the skin above the liver area, a central processing unit (10) comprising a microprocessor (7), a power source (8) and a communication device (9), and through this communication device (9) of the central unit (10) the internal sensing unit is in communication with a mobile telephone (6) with data processing and alarm function where the internal heat sensor unit (2) on the left front side of the baby underwear (1) on a center-mounted snapping garment strap (11) with a convex surface in a lower convex snap cover (5) facing the baby's body, which is attached from above by the snap connector the circular part (4) fastens to the textile strap (3) clamped between the two snap halves in such a way that the heat sensor (2) in the snap cover (5) is connected to the central unit (10) located at the bottom of the snapping garment strap (11).

The baby underwear (baby body) according to the invention allows the parent to keep the baby's night body temperature under constant monitoring and to be alerted in the event of an emergency via his mobile phone. The monitoring of body temperature, being constantly informed and alerted via mobile phone in case of emergencies definitely increase the supervising parent's sense of security during sleep at night

**List of reference numbers**
1 baby underwear (baby body)
2 heat sensor
3 lower material of textile strap
4 snap upper ring
5 snap lower cap
6 mobile phone with evaluation app
7 microprocessor
8 energy source
9 communication device
10 central unit
11 snapping garment strap

## Claims

1. Baby underwear (baby body) for measuring the continuous surface skin temperature to recognize emergencies due to an increase or decrease of temperature of a sleeping child, ***characterised by*** that the device comprises at least one metal-clad internal heat sensor unit (2) placed inside a metal-covered snap cover (5) in a direct contact with the skin above the liver area, sensing the skin temperature via a directly contacting metal housing and furthermore comprising a related central unit (10) comprising a microprocessor (7), energy supply, a power source (8) and a communication device (9) linked to and communicating with a data processing and alerting system installed on external mobile phone (6).

2. The baby underwear (baby body) according to claim 1 ***characterised by*** that the connections between the heat sensor unit (2) and the communication device (9) in the central unit (10) and the connection between the communication device in the central unit (10) and the mobile telephone (6) are wireless.

3. The baby underwear (baby body) according any of claims 1 and 2 ***characterised by*** that the wireless connection that the connections between the heat sensor unit (2) and the communication device (9) in the central unit (10) and the connection between the communication device (9) in the central unit (10) and the mobile telephone (6) is Bluetooth, preferably Bluetooth Low Energy (BLE).

4. The baby underwear (baby body) according to any of claims 1 to 3 ***characterised by*** that the heat sensor convex snap cover is in direct contact with the baby's skin above the liver area.

5. The baby underwear (baby body) according to any of claims 1 to 4 ***characterised by*** that the heat sensor snap is placed on the right side of the baby's underwear above the liver area.

6. The baby underwear (baby body) according to any of claims 1 to 5 ***characterised by**,* that a rechargeable battery is used for source of energy in the internal sensor unit.

7. The baby underwear (baby body) according to any of claims 1 to 6 ***characterised by**,* that several heat sensors may be placed on the garment's strap containing snaps.

8. The baby underwear (baby body) according to any of claims 1 to 7 ***characterised by*** that the detection of the temperature of the skin is provided through a metal cover in direct contact with the skin above the liver area, and through the communication device (9) in the central unit (10) the internal sensing unit is in communication with a mobile telephone (6) with data processing and alarm function where the internal heat sensor unit (2) on the left front side of the baby underwear (1) on a center-mounted snapping garment strap (11) with a convex surface in a lower convex snap cover (5) facing the baby's body, which is attached from above by the snap connector the circular part (4) fastens to the textile strap (3) clamped between the two snap halves in such a way that the heat sensor (2) in the snap cover (5) is connected to the central unit (10) located at the bottom of the snapping garment strap (II).
